# EUROPÄISCHE PATENTSCHRIFT

(11) **EP 3 152 573 B1**
(45) Veröffentlichungstag und Bekanntmachung des Hinweises auf die Patenterteilung: **24.10.2018**
(21) Anmeldenummer: 15747363.8
(22) Anmeldetag: 08.06.2015
(51) Int. Cl.: G01N 33/538, B01J 20/26, C08J 5/00, G01N 33/53

(54) **VERFAHREN ZUR ANALYTISCHEN AUTHENTIZITÄTSPRÜFUNG VON INHALTSSTOFFEN IN LEBENSMITTELN UNTER VERWENDUNG KÜNSTLICHER ANTIKÖRPER**
METHOD FOR ANALYTICALLY CHECKING THE AUTHENTICITY OF INGREDIENTS IN FOODSTUFFS USING ARTIFICIAL ANTIBODIES
PROCÉDÉ DE CONTRÔLE D'AUTHENTICITÉ ANALYTIQUE D'INGRÉDIENTS DANS DES ALIMENTS EN UTILISANT DES ANTICORPS ARTIFICIELS

(30) Priorität: 06.06.2014 DE 102014008135
(43) Veröffentlichungstag der Anmeldung: 12.04.2017
(73) Patentinhaber: GFL Gesellschaft Für Lebensmittel-Forschung mbH, 10787 Berlin (DE)
(72) Erfinder: HOFSOMMER, Mikko, 12101 Berlin (DE)
(74) Vertreter: Jungblut & Seuss
(86) Internationale Anmeldenummer: PCT/DE2015/000278
(87) Internationale Veröffentlichungsnummer: WO 2015/185030

(56) Entgegenhaltungen:
- WO-A1-2014/062632
- WO-A2-2007/132164
- IBRAHIM M N M ET AL: "Purification of vanillin by a molecular imprinting polymer technique", SEPARATION AND PURIFICATION TECHNOLOGY, ELSEVIER SCIENCE, AMSTERDAM, NL, Bd. 66, Nr. 3, 7. Mai 2009 (2009-05-07), Seiten 450-456, XP026052432, ISSN: 1383-5866, DOI: 10.1016/J.SEPPUR.2009.02.010 [gefunden am 2009-02-28]
- PUZIO KINGA ET AL: "Combination of computational methods, adsorption isotherms and selectivity tests for the conception of a mixed non-covalent-semi-covalent molecularly imprinted polymer of vani", ANALYTICA CHIMICA ACTA, Bd. 790, 27. Juni 2013 (2013-06-27), Seiten 47-55, XP028679659, ISSN: 0003-2670, DOI: 10.1016/J.ACA.2013.06.036
- GUO-SONG WANG ET AL: "Preparation and Recognition Properties of Vanillin-Imprinted Polymers", HELVETICA CHIMICA ACTA, Bd. 89, Nr. 12, 1. Dezember 2006 (2006-12-01), Seiten 3032-3040, XP055215703, ISSN: 0018-019X, DOI: 10.1002/hlca.200690273
- MICHAILOF ET AL: "Synthesis of caffeic acid and p-hydroxybenzoic acid molecularly imprinted polymers and their application for the selective extraction of polyphenols from olive mill waste waters", JOURNAL OF CHROMATOGRAPHY, ELSEVIER SCIENCE PUBLISHERS B.V, NL, Bd. 1182, Nr. 1, 6. Januar 2008 (2008-01-06), Seiten 25-33, XP022450401, ISSN: 0021-9673, DOI: 10.1016/J.CHROMA.2008.01.001
- DOUÉ MICKAEL ET AL: "Molecularly imprinted polymer applied to the selective isolation of urinary steroid hormones: An efficient tool in the control of natural steroid hormones abuse in cattle", JOURNAL OF CHROMATOGRAPHY, Bd. 1270, 6. November 2012 (2012-11-06), Seiten 51-61, XP028960096, ISSN: 0021-9673, DOI: 10.1016/J.CHROMA.2012.10.067
- ARMIN MOSANDL: "Mit IRMS der Natur auf der Spur", NACHRICHTEN AUS DER CHEMIE, vol. 62, no. 5, 1 May 2014 (2014-05-01), pages 517-520, XP055471038, DE ISSN: 1439-9598, DOI: 10.1002/nadc.201490156

## Beschreibung

Die vorliegende Erfindung betrifft ein Verfahren zur Abtrennung von Inhaltsstoffen aus Lebensmitteln zum Zwecke der Analytik, welches durch die Verwendung von "molecularly imprinted polymers (MIPs)", auch als künstliche Antikörper bezeichnet, gekennzeichnet ist.

Analyseverfahren zum Zwecke der Authentizitätsprüfung werden bereits im großen Umfang angewendet, z.B. um die Einhaltung lebensmittelrechtlicher Vorschriften zu prüfen. Beispielhafte Anwendungen sind die Echtheitskontrolle von Wein oder Vanille: Hierbei werden insbesondere die wertgebenden Inhaltsstoffe der Lebensmittel überprüft. Bei der Echtheitsbestimmung von Wein wird beispielsweise überprüft, ob der Zucker eines Weines aus Weintrauben stammt, oder ob der Winzer Rübenzucker oder einen anderen Zucker zugegeben hat. Bei Vanille (oder vanillehaltigen Lebensmitteln) wird der Inhaltsstoff - Vanillin - daraufhin überprüft, ob es sich um natürliches Vanillin oder um biotechnologisch hergestelltes Vanillin handelt. Bei Fruchtsäften, wie z.B.: Acerolasäften oder -marks wird die enthaltene Ascorbinsäure (Vitamin C) daraufhin untersucht, ob diese natürlichen Ursprungs ist oder ob synthetische Ascorbinsäure zugesetzt wurde.

Eine der wichtigsten Methoden für eine derartige Echtheitsbestimmung ist die Isotopenverhältnis-Massenspektroskopie (IRMS). Auf der Grundlage der Bestimmung der Isotopenverhältnisse (beispielsweise ¹H/²H, ¹³C/¹²C, ¹⁶O/¹⁷O/¹⁸O, ¹⁵N/¹⁴N) ist es möglich, festzustellen, ob die analysierten Stoffe natürlichen oder synthetischen Ursprungs sind (s. z. B. Nachrichten aus der Chemie, 62 (2014) 517f).

Ein kritischer Faktor bei der Isotopenanalytik ist die hierzu notwendige Auftrennung des Lebensmittels, wie z.B. Wein, Schokolade oder Fruchtsaft. Eine Möglichkeit hierzu besteht in der Verwendung einer ein- oder multidimensionalen Kapillar-GC, die dann mit der Isotopenmassenspektroskopie gekoppelt wird. Alternativ findet auch die präparative HPLC Anwendung. Beide Methoden sind extrem zeitaufwändig und kostenintensiv und begrenzen auf diese Weise die Probenanzahl, als auch die Anwendungsvielfalt. Die vorliegende Erfindung stellt eine vergleichsweise einfache Methode zur Verfügung, den Analyten, wie z.B. Vanillin, aus einer komplexen Naturstoffmatrix zu isolieren, um sie anschließend weiter untersuchen zu können, beispielsweise durch IRMS(s.o.), durch Cavity-ring-down-Spektroskopie (CRDS), SNIF-NMR, oder auch durch klassische NMR Spektroskopie der jeweiligen Isotope.

Erfindungsgemäß werden zu diesem Zweck "molecularly imprinted polymers (MIPs)" eingesetzt, welche auch als künstliche Antikörper bezeichnet werden. Die beiden genannten Begriffe "molecularly imprinted polymers (MIPs)" und "künstliche Antikörper" werden in diesem Dokument synonym verwendet. Derartige MIPs sind seit einigen Jahren grundsätzlich bekannt (s. z. B. Oliver Brüggemann in Synthetic polymers for biotechnology and medicine (Editor: Ruth Freitag), chapter 7, WO 98/54228, WO 94/11403).

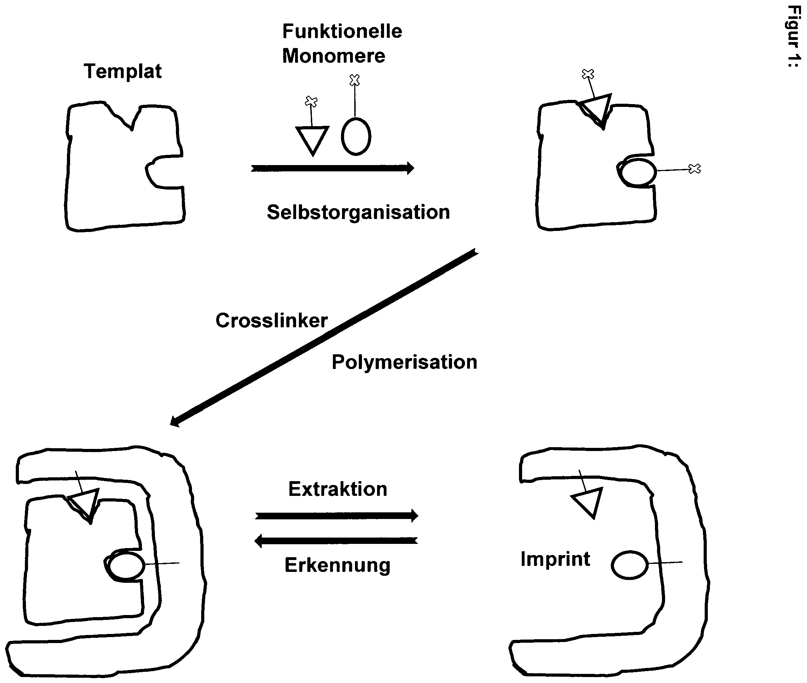

Zur Herstellung derartiger MIPs wird der gewünschte Analyt als Templat in Lösung gebracht und mit einer Mischung funktioneller Monomere versetzt. Die funktionellen Monomere binden dann in üblicher Art an entsprechende Bindungsstellen des Analyten. Die Mischung wird anschließend mit einem Crosslinker versetzt und polymerisiert. Nach Extraktion des ursprünglichen Analyten wird ein komplexes Molekül erhalten, welches den Analyten in spezifischer Weise bindet(siehe schematische Darstellung in Figur 1). Das erhaltene Molekül, das "molecularly imprinted polymer (MIP)", erkennt, nach Art eines Antikörpers, den gewünschten Analyten und ist daher in der Lage diesen aus komplexen Stoffgemischen spezifisch zu extrahieren. Optional kann das MIP die stationäre Phase einer Chromatographieeinheit (z. B. einer Chromatographiesäule) bilden. Möglich ist insbesondere eine Kartusche herzustellen, die das MIP enthält. Die zu prüfende Stoffgemisch (z.B. ein Lebensmittelauszug) wird in die Kartusche eingefüllt, wobei der Analyt von den MIPs spezifisch gebunden. wird. Nach dem Waschen kann der Analyt durch entsprechende Lösungsmittel wieder freigesetzt und der finalen Analytik (z.B. Isotopenanalytik) zugeführt werden.

Es ist natürlich auch möglich die gebildeten MIPs direkt in eine Flüssigphase zu geben und anschließend physikalisch-chemisch abzutrennen.

Eine der herausragenden Eigenschaften derartiger MIPs ist, dass dieses im Prinzip wiederverwendbar ist: Nach Erkennung des Zielmoleküls kann dieses aus dem MIP wieder entfernt werden und das MIP kann anschließend nochmals verwendet werden. Möglich ist es auch, enantioselektive MIPs herzustellen, so dass auch eine Enantiomerentrennung möglich ist. Die MIPs erreichen dabei Bindungskonstanten im Bereich natürlicher Antikörper, beispielweise im Bereich von k_{D} =10⁻⁸ - 10⁻¹⁰ M (10-0,1 nM) und sind daher von beachtlicher Selektivität. Die MIPs sind dabei sowohl gegen höhere Temperaturen (bis beispielsweise 100°C), aber auch gegen pH-Wert-Änderungen (beispielsweise pH 2-pH 12) extrem stabil, anders als beispielsweise Antikörper biologischen Ursprungs. Auf diese Weise können Trennverfahren unter Verwendung von MIPs unter anderen physikochemischen Bedingungen ausgeführt werden als mit biologischen Antikörpern.

Zur Herstellung der MIPs sind grundsätzlich Monomere geeignet, die über funktionelle Gruppen gebunden werden können. Besonders bewährt haben sich dabei Moleküle mit Ethylengruppen, die polymerisiert werden können. Folgende Monomere können beispielsweise verwendet werden Acrylsäure, Methacrylsäure, Methylmetacrylat, 1-Vinylimidazol, 4(5)-Vinylimidazol, 2-Vinylpyridin, Itaconsäure, p-Vinylbenzoesäure, Ethylstyrol, (2-Metacrylamido)-6-Methylpyridin, 2-Acrylamido-2-Methyl-1-Propan-Sulfonsäure, p-Vinylphenylboronsäure, p-Vinylbenzyl-Amin, 2-(p-Vinylphenyl)-1,3-propandiol, p-Vinylbenzylalkohol, p-Vinylphenylcarbonyl, p-Vinylbenzoesäure.

Je nach Art des Monomers kann dieses nicht-kovalent oder kovalent an das jeweilige Templat binden. Je nach Templat kann nur eine Substanz als Monomer verwendet werden, es können aber auch zwei, drei oder mehr verschiedene Monomere verwendet werden.

Als Crosslinker zur Herstellung der MIPs werden beispielsweise Ethylenglycoldimethacrylat (EGDMA), Paradivinylbenzol (p-DVB), N,N'-methylendiacrylamid, N,N'-1,4-phenylendiacrylamid, N,O-bisacrylol-L-phenylalaninol, Trimethylolpropan Trimethacrylat (TRIM), Pentaerythritol Triacrylat und Pentaerythritol Tetraacrylat verwendet.

### Herstellung von MIPs

Die Herstellung derartiger MIPs ist in der Literatur beschrieben. Grundsätzlich wird der Prozess folgendermaßen ablaufen:
1. Lösung des Analyten in einem geeigneten Lösungsmittel, wie beispielsweise DMF, DMSO, ACN, THF oder Chloroform.
2. Zugabe eines Monomeren oder einer einer Mischung verschiedener Monomere und des Crosslinkers.
3. Zugabe eines Polymerisationsstarters.
4. Polymerisation unter Einwirkung von UV-Licht oder Wärme.

Die gebildeten MIPs fallen üblicherweise aus der Lösung aus, so dass sie leicht abgetrennt werden können.

### Anwendungsbeispiel von MIPs

Unter Verwendung derartiger MIPs kann beispielsweise Vanillin sehr einfach aus einem komplexen Lebensmittelprodukt, wie beispielsweise Schokolade abgetrennt werden. Hierzu wird die zerkleinerte feste Probe in Wasser aufgeschlämmt, gegebenenfalls mit Methanol versetzt und je nach Matrix filtriert / zentrifugiert. Anschließend wird der erhaltene Extrakt direkt über eine mit dem analytspezischen MIP gefüllte Kartusche gegeben.

Das MIP Material mit dem Vanillin wird mit geeigneten Lösemitteln z.B. Diethylether und Pufferlösung z.B. Natriumdicarbonat gewaschen. Anschließend wird die Kartusche unter leichtem Vakuum trocken gesaugt. Im letzten Schritt wird das Vanillin mit einem geeigneten Lösungsmittel z.B. Methanol eluiert. Das Eluat wird unter Stickstoff getrocknet, gegebenenfalls auch einer Gefriertrocknung unterworfen, so dass Vanillin in hoher Reinheit > 90% als Feststoff gewonnen wird.

Anschließend kann das Vanillin ohne weiteres umgehend der IRMS, der CRDS oder einer Isotopen-NMR Analytik zugeführt werden. In manchen Fällen empfiehlt es sich, den gewünschten Analyten aus dem Lebensmittel durch bestimmte Vorverfahren (Auszugsverfahren) zu extrahieren, wie beispielsweise durch Wasserdampfdestillation oder Herstellung eines alkoholischen Auszugs. Der gewonnene Auszug (Extrakt) kann dann, wie oben prinzipiell beschrieben - gegebenfalls nach einer weiteren Einengung - mit den MIPS versetzt und dann wie ebenfalls prinzipiell oben beschrieben weiter verarbeitet werden.

In prinzipiell gleicher Weise können andere Analyten aus Lebensmitteln, deren Roh- und Halbwaren sowie deren Zutaten (wie beispielweise Aromen) abgetrennt und der Analytik unterzogen werden. Zur sprachlichen Vereinfachung werden in diesem Dokument mit dem Begriff "Lebensmittel" auch derartige Roh- und Halbwaren und Zutaten umfasst.

Als mögliche Lebensmittel beispielsweise genannt seien: Frucht-/Gemüsesaft sowie daraus gewonnene Erzeugnisse (z.B. Mark, Konzentrate, Markkonzentrate, Nektare, Getränke), Erfrischungsgetränke, alkoholische Getränke, Wein, Spirituosen, Bier, Süßwaren, Schokolade, Honig, Speiseeis, Marmelade, Konfitüre, Fisch-/Fleisch sowie hieraus gewonnene Erzeugnisse und Säuglingsnahrung. Ferner seien Aromastoffe, Aromastoffgemische und deren Zusammensetzungen genannt. Bevorzugte Lebensmittel sind Frucht-/Gemüsesaft sowie deren Erzeugnisse.

Als mögliche Analyte beispielsweise genannt seien: Saccharose, Glucose oder Fructose, Maltose, Sorbit, Vanillin, fruchttypisch wertgebende Aromastoffe wie z.B. Benzaldehyd, Ethylbutyrat, gamma-Octalactone, delta-Decalactone, gamma-Decalactone, trans-2-Hexenal, 1-Hexanol, trans-2-Hexanol, Hexylacetat, Ethyl-3-(methylthio)-propionat, Methyl-3-(methylthio)-propionat, alpha-Ionon, Ethyl-2,4-decadienoat, Hexylhexanoate, Nootkatone, Decanal, Furaneol (4-Hydroxy-2,5-dimethyl-3(2H)furanon), Mesifuran (2,5-dimethyl-4-methoxy-3(2H)furanon), Ascorbinsäure, Dehydroascorbinsäure, Äpfelsäure, Citronensäure, Iso-Citronensäure, Weinsäure, Aminosäuren, Glycerin, Phosphat und Koffein.
Besonders bevorzugte Analyte sind Vanillin, Ascorbinsäure, Saccharose, Glucose und Fructose.
Zur Probenvorbereitung können die o.g. Stoffe auch derivatisiert werden, beispielsweise durch Einführung von Schutzgruppen. Diese Derivatisierung dient insbesondere der Verhinderung von Isotopenaustauch während des Analytikprozesses. Eine weitere Anwendung der Derivatisierung ist beispielsweise die Verbesserung der Trennschärfe bei chemisch sehr ähnlichen Molekülen (z.B. Glucose/Fructose) durch gezielte Derivatisierung einer Komponente.

Wie oben bereits ausgeführt können MIPs auch die stationäre Phase einer Chromatographiesäule bilden. In diesem Fall wird die zu untersuchende Lösung einfach auf die Säule aufgetragen und mit einem ersten Lösungsmittel durch die Säule gespült. Der durch die Verwendung des MIPs auf der Säule festgehaltene Analyt wird anschließend unter Verwendung eines anderen Lösungsmittels wieder aus dem MIP abgetrennt und liegt anschließend in nahezu reiner Form vor.

Weiterhin ist es möglich eine Kartusche herzustellen, die das MIP enthält, in Analogie zur "Solid Phase Extraction (SPE)". Geeignet sind hierzu vor allem Kunststoffkartuschen, welche typischerweise 5-20 ml Fassungsvermögen aufweisen. Das zu prüfende Stoffgemisch (z.B. ein Lebensmittelauszug) wird in die Kartusche eingefüllt, wobei der Analyt von den MIPs spezifisch gebunden wird. Nach dem Waschen kann der Analyt durch entsprechende Lösungsmittel wieder freigesetzt und der finalen Analytik (z.B. Isotopenanalytik) zugeführt werden. Gewünschtenfalls kann die Einfüllung einer Probe und die spätere Analytik auch an verschiedenen Orten erfolgen: Beispielsweise kann die Probennahme und Einfüllung in die Kartusche in einem lebensmittelverarbeitenden Betrieb erfolgen. Die verschlossene Kartusche kann dann in ein Labor versandt werden, welches die weitere Aufarbeitung und die abschließende Isotopenanalytik durchführt.

### Beispiele

Das erfindungsgemäße Verfahren wird durch die nachfolgenden Beispiele noch näher beschrieben, ohne dass diese einschränkend wirken sollen.

### A.Herstellungsbeispiel für ein Vanillin MIP

Vanillin wird in Ethyl^{-*t*-}Butylether gelöst. Die Lösung wird mit mindestens 3 Monomeren versetzt, welche aus folgender Liste ausgewählt sind: Acrylsäure, Methacrylsäure, Methylmetacrylat, 1-Vinylimidazol, 4(5)-Vinylimidazol, 2-Vinylpyridin, Itaconsäure, p-Vinylbenzoesäure, Ethylstyrol, (2-Metacrylamido)-6-Methylpyridin, 2-Acryl-amido-2-Methyl-1-Propan-Sulfonsäure, p-Vinylphenylboronsäure, p-Vinylbenzyl-Amin, 2-(p-Vinylphenyl)-1,3-propandiol, p-Vinylbenzylalkohol, p-Vinylphenylcarbonyl, p-Vinylbenzoesäure. Die entstandene Lösung wird mit mindestens einem Crosslinker versetzt, wobei der Crosslinker ausgewählt wird aus folgender Liste: Ethylenglycoldimethacrylat (EGDMA), Paradivinylbenzol (p-DVB), N,N'-methylendiacrylamid, N,N'-1,4-phenylendiacrylamid, N,O-bisacrylol-L-phenylalaninol, Trimethylolpropan Trimethacrylat (TRIM), Pentaerythritol Triacrylat und Pentaerythritol Tetraacrylat.

Nach Zugabe einer kleinen Menge von AIBN als Polymerisationsstarter wird die Lösung 4 Stunden unter UV-Licht gerührt. Dabei fallen die gebildeten MIPs, beladen mit Vanillin, aus der Lösung aus. Sie werden abgetrennt, mit wenig Wasser gewaschen und im Vakuum getrocknet. Aus den abgetrennten MIPs wird das Vanillin in klassischer Weise durch Wasser/Methanol eluiert. Die Eluierung wird solange vorgenommen, bis kein Vanillin mehr nachweisbar ist. Weitere Details der Bildung von MIPs können der Literatur entnommen werden (s. z. B. WO 94/11403 oder WO 98/54228 sowie hierin zitierte Literatur).

### B. Nachweis biotechnologisch hergestellter Ascorbinsäure in Acerolamark

Eine Acerolamarkprobe wird im Bedarfsfall matrixabhängig enzymatisch (z.B. Pektinasen) vorbehandelt und filtriert oder zentrifugiert. Das Filtrat wird direkt mit den analytspezischen MIP in Kontakt gebracht, indem es über eine mit dem MIP gefüllte Kartusche gegeben wird. Das MIP Material (Herstellung und Zusammensetzung in Analogie zu Bsp. A) mit der Ascorbinsäure wird anschließend mit geeigneten Lösemitteln z.B. Diethylether gewaschen. Anschließend wird die Kartusche unter leichtem Vakuum trocken gesaugt. Im letzten Schritt wird die Ascorbinsäure mit einer geeigneten Pufferlösung z.B. Acetatpufferlösung eluiert, wobei eine pH-Wert Änderung hervorgerufen wird und die Ascorbinsäure deprotoniert vorliegt. Das Eluat wird unter Stickstoff getrocknet, gegebenenfalls auch einer Gefriertrocknung unterworfen, so dass ca. 1 mg Ascorbinsäure mit ausreichender Reinheit > 90 % als Feststoff gewonnen wird.

Anschließend kann die Probe der SNIF-NMR Analytik zugeführt werden.

### C. Nachweis von nicht genuinem Benzaldehyd in Kirschnektaren

Zur Isolierung von Benzaldehyd in Kirschnektar ist eine Anreicherung notwendig. Hierzu wird die Probe mittels SDE (Simultane Wasserdampfdestillation und Extraktion) aufgearbeitet und an einer Vigreux Kolonne weiter konzentriert. Der erhaltene Extrakt wird anschließend über eine mit dem analytspezischen MIP (Herstellung und Zusammensetzung in Analogie zu Bsp. A) gefüllte Kartusche gegeben.

Das MIP Material mit dem Benzaldehyd wird mit geeigneten Lösemitteln z.B. ^{*tert*.*-*}Butylether und Pufferlösung z.B. Natriumdicarbonat gewaschen. Anschließend wird die Kartusche unter leichtem Vakuum trocken gesaugt. Im letzten Schritt wird das Benzaldehyd mit einem geeigneten Lösungsmittel z.B. Pentan eluiert. Das Eluat wird unter Stickstoff getrocknet, gegebenenfalls auch einer Gefriertrocknung unterworfen, so dass Benzaldehyd als Reinstsubstanz gewonnen wird.

Anschließend kann die Probe der Isotopenanalytik mittels Cavity-ring-down-Spektroskopie (CRDS) zugeführt werden.

## Patentansprüche

1. Verfahren zur Authentizitätsprüfung eines Analyten aus einem Lebensmittel, **dadurch gekennzeichnet, dass**
a) die Extraktion des gewünschten Analyten aus dem Lebensmittel oder einem Lebensmittelauszug durch molecularly imprinted polymers (MIPs) erfolgt,
dass
b) die mit dem Analyten beladenen MIPS abgetrennt werden,
dass
c) aus den mit dem Analyten beladenen MIPs der Analyt wieder entfernt wird
und dass
d) der auf diese Weise extrahierte Analyt einer Isotopenverhältnis-Massenspektroskopie (IRMS) oder einer Cavity-ring-down-Spektroskopie (CRDS) zur Bestimmung des Isotopenverhältnisses, ausgewählt aus ¹H/²H, ¹³C/¹²C, ¹⁶O/¹⁷O/¹⁸O und/oder ¹⁵N/¹⁴N, zugeführt wird.

2. Verfahren gemäß Anspruch 1, **dadurch gekennzeichnet, dass** die Extraktion des Analyten so erfolgt, dass sich die MIPs in einer Kartusche befinden.

3. Verfahren gemäß Anspruch 1, **dadurch gekennzeichnet, dass** der Analyt Saccharose, Glucose, Fructose, Maltose, Sorbit, Vanillin, Benzaldehyd, Ethylbutyrat, gamma-Octalactone, delta-Decalactone, gamma-Decalactone, trans-2-Hexenal, 1-Hexanol, trans-2-Hexanol, Hexylacetat, Ethyl-3-(methylthio)-propionat, Methyl-3-(methylthio)-propionat, alpha-Ionon, Ethyl-2,4-decadienoat, Hexylhexanoate, Nootkatone, Decanal, Furaneol (4-Hydroxy-2,5-dimethyl-3(2H)furanon), Mesifuran (2,5-dimethyl-4-methoxy-3(2H)furanon), Ascorbinsäure, Dehydroascorbinsäure, Äpfelsäure, Citronensäure, Iso-Citronensäure, Weinsäure, Aminosäuren, Glycerin, Phosphat, Koffein oder ein Derivat dieser Stoffe ist.

4. Verfahren gemäß Anspruch 3, **dadurch gekennzeichnet, dass** der Analyt Vanillin, Ascorbinsäure, Saccharose, Glucose und Fructose oder ein Derivat dieser Stoffe ist.

5. Verfahren gemäß Anspruch 1, **dadurch gekennzeichnet, dass** das Lebensmittel ausgewählt ist aus folgender Liste:
Frucht-/Gemüsesaft sowie daraus gewonnene Erzeugnisse,
Erfrischungsgetränk,
alkoholisches Getränk,
Wein,
Spirituose,
Bier,
Süßware,
Schokolade,
Honig,
Speiseeis,
Marmelade,
Konfitüre,
Säuglingsnahrung,
Fisch-/Fleisch sowie daraus gewonnene Erzeugnisse.

6. Verfahren gemäß Anspruch 5, **dadurch gekennzeichnet, dass** das Lebensmittel Frucht-/Gemüsesaft oder ein daraus gewonnenes Erzeugnis ist.

7. Verwendung eines Molecularly imprinted Polymer (MIP), bestehend aus einem Polymer, welches unter Verwendung eines Lebensmittelinhaltsstoffes als molekulares Target hergestellt wurde, wobei mindestens einer der folgenden Substanzen
Acrylsäure, Methacrylsäure, Methylmetacrylat, 1-Vinylimidazol, 4(5)-Vinylimidazol, 2-Vinylpyridin, Itaconsäure, p-Vinylbenzoesäure, Ethylstyrol, (2-Metacrylamido)-6-Methylpyridin, 2-Acrylamido-2-Methyl-1-Propan-Sulfonsäure, p-Vinylphenylboronsäure, p-Vinylbenzyl-Amin, 2-(p-Vinylphenyl)-1,3-propandiol, p-Vinylbenzylalkohol, p-Vinylphenylcarbonyl, p-Vinylbenzoesäure
als Monomer verwendet wurden und wobei mindestens einer der folgenden Stoffe Ethylenglycoldimethacrylat (EGDMA), Paradivinylbenzol (p-DVB), N,N'-methylendiacrylamid, N,N'-1,4-phenylendiacrylamid, N,O-bisacrylol-L-phenylalaninol, Trimethylolpropan Trimethacrylat (TRIM), Pentaerythritol Triacrylat und Pentaerythritol Tetraacrylat
als Crosslinker verwendet wurde,
**dadurch gekennzeichnet, dass**
das MIP selektiv den ursprünglich eingesetzten Lebensmittelinhaltsstoff als molekulares Target bindet,
zur Durchführung eines Verfahrens zur Authentizitätsbestimmung eines Analyten aus einem Lebensmittel gemäß Anspruch 1.

8. Verwendung eines Molecularly imprinted Polymer (MIP), gemäß Anspruch 7, **dadurch gekennzeichnet, dass** mindestens zwei der folgenden Substanzen Acrylsäure, Methacrylsäure, Methylmetacrylat, 1-Vinylimidazol, 4(5)-Vinylimidazol, 2-Vinylpyridin, Itaconsäure, p-Vinylbenzoesäure, Ethylstyrol, (2-Metacrylamido)-6-Methylpyridin, 2-Acrylamido-2-Methyl-1-Propan-Sulfonsäure, p-Vinylphenylboronsäure, p-Vinylbenzyl-Amin, 2-(p-Vinylphenyl)-1,3-propandiol, p-Vinylbenzylalkohol, p-Vinylphenylcarbonyl, p-Vinylbenzoesäure
als Monomer verwendet wurden.

9. Verwendung eines Molecularly imprinted Polymer (MIP), gemäß Anspruch 7, **dadurch gekennzeichnet, dass** das Target
Saccharose, Glucose oder Fructose, Maltose, Sorbit, Vanillin, Benzaldehyd, Ethylbutyrat, gamma-Octalactone, delta-Decalactone, gamma-Decalactone, trans-2-Hexenal, 1-Hexanol, trans-2-Hexanol, Hexylacetat, Ethyl-3-(methylthio)-propionat, Methyl-3-(methylthio)-propionat, alpha-Ionon, Ethyl-2,4-decadienoat, Hexylhexanoate, Nootkatone, Decanal, Furaneol (4-Hydroxy-2,5-dimethyl-3(2H)furanon), Mesifuran (2,5-dimethyl-4-methoxy-3(2H)furanon), Ascorbinsäure, Dehydroascorbinsäure, Äpfelsäure, Citronensäure, Iso-Citronensäure, Weinsäure, Aminosäuren, Glycerin, Phosphat, Koffein, ist, oder in einer derivatisierten Form vorliegt.

10. Vorgefüllte Kartusche, enthaltend mindestens ein Molecularly imprinted Polymer (MIP), bestehend aus einem Polymer, welches unter Verwendung eines Lebensmittelinhaltsstoffes als molekulares Target hergestellt wurde, wobei mindestens einer der folgenden Substanzen
Acrylsäure, Methacrylsäure, Methylmetacrylat, 1-Vinylimidazol, 4(5)-Vinylimidazol, 2-Vinylpyridin, Itaconsäure, p-Vinylbenzoesäure, Ethylstyrol, (2-Metacrylamido)-6-Methylpyridin, 2-Acrylamido-2-Methyl-1-Propan-Sulfonsäure, p-Vinylphenylboronsäure, p-Vinylbenzyl-Amin, 2-(p-Vinylphenyl)-1,3-propandiol, p-Vinylbenzylalkohol, p-Vinylphenylcarbonyl, p-Vinylbenzoesäure
als Monomer verwendet wurden und wobei mindestens einer der folgenden Stoffe Ethylenglycoldimethacrylat (EGDMA), Paradivinylbenzol (p-DVB), N,N'-methylendiacrylamid, N,N'-1,4-phenylendiacrylamid, N,O-bisacrylol-L-phenylalaninol, Trimethylolpropan Trimethacrylat (TRIM), Pentaerythritol Triacrylat und Pentaerythritol Tetraacrylat
als Crosslinker verwendet wurde,
wobei das MIP selektiv den ursprünglich eingesetzten Lebensmittelinhaltsstoff als molekulares Target bindet, zur Verwendung in einem Verfahren zur Authentizitätsprüfung eines Analyten aus einem Lebensmittel durch Isotopenverhältnis-Massenspektroskopie (IRMS) oder einer Cavity-ring-down-Spektroskopie (CRDS) zur Bestimmung des Isotopenverhältnisses, ausgewählt aus ¹H/²H, ¹³C/¹²C, ¹⁶O/¹⁷O/¹⁸O und/oder ¹⁵N/¹⁴N.

11. Vorgefüllte Kartusche, gemäß Anspruch 10, **dadurch gekennzeichnet, dass** mindestens zwei der folgenden Substanzen
Acrylsäure, Methacrylsäure, Methylmetacrylat, 1-Vinylimidazol, 4(5)-Vinylimidazol, 2-Vinylpyridin, Itaconsäure, p-Vinylbenzoesäure, Ethylstyrol, (2-Metacrylamido)-6-Methylpyridin, 2-Acrylamido-2-Methyl-1-Propan-Sulfonsäure, p-Vinylphenylboronsäure, p-Vinylbenzyl-Amin, 2-(p-Vinylphenyl)-1,3-propandiol, p-Vinylbenzylalkohol, p-Vinylphenylcarbonyl, p-Vinylbenzoesäure
als Monomer für die Herstellung des MIP verwendet wurden.

12. Vorgefüllte Kartusche, gemäß Anspruch 10, **dadurch gekennzeichnet, dass** das Target
Saccharose, Glucose oder Fructose, Maltose, Sorbit, Vanillin, Benzaldehyd, Ethylbutyrat, gamma-Octalactone, delta-Decalactone, gamma-Decalactone, trans-2-Hexenal, 1-Hexanol, trans-2-Hexanol, Hexylacetat, Ethyl-3-(methylthio)-propionat, Methyl-3-(methylthio)-propionat, alpha-Ionon, Ethyl-2,4-decadienoat, Hexylhexanoate, Nootkatone, Decanal, Furaneol (4-Hydroxy-2,5-dimethyl-3(2H)furanon), Mesifuran (2,5-dimethyl-4-methoxy-3(2H)furanon), Ascorbinsäure, Dehydroascorbinsäure, Äpfelsäure, Citronensäure, Iso-Citronensäure, Weinsäure, Aminosäuren, Glycerin, Phosphat, Koffein, ist, oder in einer derivatisierten Form vorliegt.

## Claims

1. A method for authenticity testing of an analyte from a foodstuff, **characterized by** that
a) the extraction of the desired analyte from the foodstuff or a food extract takes place by molecularly imprinted polymers (MIPs), that
b) the MIPS laden with the analyte are separated, that
c) from the MIPs laden with the analyte, the analyte is removed again and that
d) the thus extracted analyte is subjected to an isotope-ratio mass spectroscopy (IRMS) or a cavity ring-down spectroscopy (CRDS) for determining the isotope ratio, selected from ¹H/²H, ¹³C/¹²C, ¹⁶O/¹⁷O/¹⁸O and/or ¹⁵N/¹⁴N.

2. The method according to claim 1, **characterized by** that the extraction of the analyte takes place such that the MIPs are located in a cartridge.

3. The method according to claim 1, **characterized by** that the analyte is saccharose, glucose, fructose, maltose, sorbitol, vanillin, benzaldehyde, ethyl butyrate, gamma-octalactone, delta-decalactone, gamma-decalactone, trans-2-hexenal, 1-hexanol, trans-2-hexanol, hexyl acetate, ethyl-3-(methylthio)-propionate, methyl-3-(methylthio)-propionate, alpha-ionone, ethyl-2,4-decadienoate, hexyl hexanoate, nootkatone, decanal, furaneol-(4-hydroxy-2,5-dimethyl-3(2H)furanone), mesifuran(2,5-dimethyl-4-methoxy-3(2H) furanone), ascorbic acid, dehydroascorbic acid, malic acid, citric acid, isocitric acid, tartaric acid, amino acids, glycerol, phosphate, caffeine or a derivative of these substances.

4. The method according to claim 3, **characterized by** that the analyte is vanillin, ascorbic acid, saccharose, glucose, fructose or a derivative of these substances.

5. The method according to claim 1, **characterized by** that the foodstuff is selected from the following list:
fruit/vegetable juice and products obtained therefrom,
soft drink,
alcoholic beverage,
wine,
spirit,
beer,
confectionery,
chocolate,
honey,
ice cream,
marmalade,
jam,
baby food,
fish/meat and products obtained therefrom.

6. The method according to claim 5, **characterized by** that the foodstuff is fruit/vegetable juice or a product obtained therefrom.

7. Use of a molecularly imprinted polymer (MIP), comprising a polymer, which has been produced using a foodstuff ingredient as a molecular target, at least one of the following substances
acrylic acid, methacrylic acid, methyl methacrylate, 1-vinylimidazole, 4(5)-vinylimidazole, 2-vinylpyridine, itaconic acid, p-vinyl benzoic acid, ethylstyrene, (2-methacrylamido)-6-methylpyridine, 2-acrylamido-2-methyl-1-propane sulfonic acid, p-vinylphenyl boronic acid, p-vinylbenzylamine, 2-(p-vinylphenyl)-1,3-propandiol, p-vinylbenzyl alcohol, p-vinylphenyl carbonyl, p-vinyl benzoic acid
being used as a monomer, and at least one of the following substances
ethylene glycol dimethacrylate (EGDMA), paradivinylbenzene (p-DVB), N,N'-methylenediacrylamide, N,N'-1,4-phenylenediacrylamide, N,O-bis-acrylol-L-phenylalaninol, trimethylolpropane trimethacrylate (TRIM), pentaerythritol triacrylate and pentaerythritol tetraacrylate being used as a crosslinker,
**characterized by** that
the MIP selectively binds to the originally used foodstuff ingredient as a molecular target, for carrying-out a method for determining the authenticity of an analyte from a foodstuff according to claim 1.

8. The use of a molecularly imprinted polymer (MIP) according to claim 7, **characterized by** that at least two of the following substances acrylic acid, methacrylic acid, methyl methacrylate, 1-vinylimidazole, 4(5)-vinylimidazole, 2-vinylpyridine, itaconic acid, p-vinyl benzoic acid, ethylstyrene, (2-methacrylamido)-6-methylpyridine, 2-acrylamido-2-methyl-1-propane sulfonic acid, p-vinylphenyl boronic acid, p-vinylbenzylamine, 2-(p-vinylphenyl)-1,3-propandiol, p-vinylbenzyl alcohol, p-vinylphenyl carbonyl, p-vinyl benzoic acid
are used as monomers.

9. The use of a molecularly imprinted polymer (MIP) according to claim 7, **characterized by** that the target is saccharose, glucose or fructose, maltose, sorbitol, vanillin, benzaldehyde, ethyl butyrate, gamma-octalactone, delta-decalactone, gamma-decalactone, trans-2-hexenal, 1-hexanol, trans-2-hexanol, hexyl acetate, ethyl 3-(methylthio)-propionate, methyl 3-(methylthio)-propionate, alpha-ionone, ethyl-2,4-decadienoate, hexyl hexanoate, nootkatone, decanal, furaneol(4-hydroxy-2,5-dimethyl-3(2H) furanone), mesifuran(2,5-dimethyl-4-methoxy-3(2H)furanone), ascorbic acid, dehydroascorbic acid, malic acid, citric acid, isocitric acid, tartaric acid, amino acids, glycerol, phosphate, caffeine, or is present in a derivatized form.

10. A pre-filled cartridge, including at least one molecularly imprinted polymer (MIP), comprising a polymer, which has been produced using a foodstuff ingredient as a molecular target, at least one of the following substances acrylic acid, methacrylic acid, methyl methacrylate, 1-vinylimidazole, 4(5)-vinylimidazole, 2-vinylpyridine, itaconic acid, p-vinyl benzoic acid, ethylstyrene, (2-methacrylamido)-6-methylpyridine, 2-acrylamido-2-methyl-1-propane sulfonic acid, p-vinylphenyl boronic acid, p-vinylbenzylamine, 2-(p-vinylphenyl)-1,3-propandiol, p-vinylbenzyl alcohol, p-vinylphenyl carbonyl, p-vinyl benzoic acid
being used as a monomer, and at least one of the following substances
ethylene glycol dimethacrylate (EGDMA), paradivinylbenzene (p-DVB), N,N'-methylenediacrylamide, N,N'-1,4-phenylenediacrylamide, N,O-bis-acrylol-L-phenylalaninol, trimethylolpropane trimethacrylate (TRIM), pentaerythritol triacrylate and pentaerythritol tetraacrylate being used as a crosslinker,
wherein the MIP selectively binds to the originally foodstuff ingredient as a molecular target, for use in a method for authenticity testing of an analyte from a foodstuff by isotope-ratio mass spectroscopy (IRMS) or a cavity ring-down spectroscopy (CRDS) for determining the isotope ratio, selected from ¹H/²H, ¹³C/¹²C, ¹⁶O/¹⁷O/¹⁸O and/or ¹⁵N/¹⁴N.

11. The pre-filled cartridge according to claim 10, **characterized by** that at least two of the following substances
acrylic acid, methacrylic acid, methyl methacrylate, 1-vinylimidazole, 4(5)-vinylimidazole, 2-vinylpyridine, itaconic acid, p-vinyl benzoic acid, ethylstyrene, (2-methacrylamido)-6-methylpyridine, 2-acrylamido-2-methyl-1-propane sulfonic acid, p-vinylphenyl boronic acid, p-vinylbenzylamine, 2-(p-vinylphenyl)-1,3-propandiol, p-vinylbenzyl alcohol, p-vinylphenyl carbonyl, p-vinyl benzoic acid
have been used as monomers for producing the MIP.

12. The pre-filled cartridge according to claim 10, **characterized by** that the target is saccharose, glucose or fructose, maltose, sorbitol, vanillin, benzaldehyde, ethyl butyrate, gamma-octalactone, delta-decalactone, gamma-decalactone, trans-2-hexenal, 1-hexanol, trans-2-hexanol, hexyl acetate, ethyl 3-(methylthio)-propionate, methyl 3-(methylthio)-propionate, alpha-ionone, ethyl-2,4-decadienoate, hexyl hexanoate, nootkatone, decanal, furaneol(4-hydroxy-2,5-dimethyl-3(2H)furanone), mesifuran(2,5-dimethyl-4-methoxy-3(2H) furanone), ascorbic acid, dehydroascorbic acid, malic acid, citric acid, isocitric acid, tartaric acid, amino acids, glycerol, phosphate, caffeine, or is present in a derivatized form.

## Revendications

1. Procédé de contrôle d'authenticité d'un analyte à partir d'un aliment, **caractérisé en ce que**
a) l'extraction de l'analyte désiré à partir de l'aliment ou d'un extrait d'un aliment a lieu par des polymères à empreinte moléculaire (MIPs), que
b) les MIPS chargés de l'analyte sont séparés, que
c) à partir des MIPs chargés de l'analyte, l'analyte est enlevé et que
d) l'analyte ainsi extrait est soumis à une spectroscopie de masse isotopique (IRMS) ou une spectroscopie à cavité optique (CRDS) pour déterminer le ratio isotopique, choisi à partir de ¹H/²H, ¹³C/¹²C, ¹⁶O/¹⁷O/¹⁸O et/ou ¹⁵N/¹⁴N.

2. Procédé selon la revendication 1, **caractérisé en ce que** l'extraction de l'analyte a lieu de façon que les MIPs se trouvent dans une cartouche.

3. Procédé selon la revendication 1, **caractérisé en ce que** l'analyte est du saccharose, du glucose, du fructose, du maltose, du sorbitol, de la vanilline, du benzaldéhyde, du butanoate d'éthyle, de la gamma-octalactone, de la delta-décalactone, de la gamma-décalactone, du trans-2-hexénal, du 1-hexanol, du trans-2-hexanol, de l'acétate d'hexyle, du 3-(méthylthio)-propionate d'éthyle, du 3-(méthylthio)-propionate de méthyle, de l'alpha-ionone, du 2,4-décadiénoate d'éthyle, du hexanoate d'hexyle, du nootkatone, du décanal, de la 3(2H)-furanone de furanéol-(4-hydroxy-2,5-diméthyle), de la 3(2H)-furanone de mésifurane (2,5-diméthyle-4-méthoxy), de l'acide ascorbique, de l'acide déshydroascorbique, de l'acide malique, de l'acide citrique, de l'acide isocitrique, de l'acide tartarique, des acides aminés, du glycérol, du phosphate, de la caféine ou un dérivé de ces substances.

4. Procédé selon la revendication 3, **caractérisé en ce que** l'analyte est de la vanilline, de l'acide ascorbique, du saccharose, du glucose, du fructose ou un dérivé de ces substances.

5. Procédé selon la revendication 1, **caractérisé en ce que** l'aliment est choisi à partir de la liste suivante:
du jus de fruit/légumes et des produits obtenus de ceux-ci,
du boisson rafraîchissante,
du boisson alcoolique,
du vin,
des spiritueux,
de la bière,
de la confiserie,
du chocolat,
du miel,
de la glace,
de la marmelade,
de la confiture,
de la nourriture pour bébés,
du poisson/de la viande et des produits obtenus de ceux-ci.

6. Procédé selon la revendication 5, **caractérisé en ce que** l'aliment est du jus de fruit/légumes ou un produits obtenu de ceux-ci.

7. Utilisation d'un polymère à empreinte moléculaire (MIP), comprenant un polymère, qui a été produit en utilisant un ingrédient d'aliment comme cible moléculaire, au moins une des substances suivantes
de l'acide acrylique, de l'acide méthacrylique, du méthacrylate de méthyle, du 1-vinylimidazole, du 4(5)-vinylimidazole, de la 2-vinylpyridine, de l'acide itaconique, de l'acide benzoïque de p-vinyle, de l'éthylstyrène, (2-méthacrylamido)-6-méthylpyridine, de l'acide 2-acrylamido-2-méthyle-1-propanesulfonique, de l'acide p-vinylphénylboronique, du p-vinylbenzylamine, du 2-(p-vinylphényle)-1,3-propandiol, p-vinylbenzylalcool, p-vinylphénylcarbonyl, de l'acide benzoïque de p-vinyle
étant utilisée comme monomère, et au moins une des substances suivantes
du diméthacrylate d'éthylène glycol (EGDMA), du para-divinylbenzène (p-DVB), du N,N'-méthylènediacrylamide, du N,N'-1,4-phénylènediacrylamide, du N,O-bisacrylol-L-phénylalaninol, du triméthacrylate de triméthylolpropane (TRIM), du triacrylate de pentaérythritol et due tétraacrylate de pentaérythritol
étant utilisée comme crosslinker,
**caractérisé en ce que**
le MIP sélectivement lie à l'ingrédient d'aliment utilisé à l'origine comme cible moléculaire, pour l'exécution d'un procédé pour déterminer l'authenticité d'un analyte à partir d'un aliment selon la revendication 1.

8. Utilisation d'un polymère à empreinte moléculaire (MIP) selon la revendication 7, **caractérisé en ce qu'**au moins deux des substances suivantes
de l'acide acrylique, de l'acide méthacrylique, du méthacrylate de méthyle, du 1-vinylimidazole, du 4(5)-vinylimidazole, de la 2-vinylpyridine, de l'acide itaconique, de l'acide benzoïque de p-vinyle, de l'éthylstyrène, de la (2-méthacrylamido)-6-méthylpyridine, de l'acide 2-acrylamido-2-méthyle-1-propanesulfonique, de l'acide p-vinylphénylboronique, du p-vinylbenzylamine, du 2-(p-vinylphényle)-1,3-propandiol, du p-vinylbenzylalcool, du p-vinylphénylcarbonyl, de l'acide benzoïque de p-vinyle sont utilisées comme des monomères.

9. Utilisation d'un polymère à empreinte moléculaire (MIP) selon la revendication 7, **caractérisé en ce que** la cible est
du saccharose, du glucose ou du fructose, du maltose, du sorbitol, de la vanilline, du benzaldéhyde, du butyrate d'éthyle, de la gamma-octalactone, de la delta-décalactone, de la gamma-décalactone, du trans-2-hexénal, du 1-hexanol, du trans-2-hexanol, de l'acétate d'hexyle, du 3-(méthylthio)-propionate d'éthyle, du 3-(méthylthio)-propionate de méthyle, de l'alpha-ionone, du 2,4-décadiénoate d'éthyle, du hexanoate d'hexyle, du nootkatone, du décanal, du 3(2H) furanone de furanéol (4-hydroxy-2,5-diméthyle), du 3(2H) furanone de mésifurane (2,5-diméthyle-4-méthoxy), de l'acide ascorbique, de l'acide déshydroascorbique, de l'acide malique, de l'acide citrique, de l'acide isocitrique, de l'acide tartarique, des acides aminés, du glycérol, du phosphate, de la caféine, ou est présent en forme dérivée.

10. Cartouche préremplie, comportant au moins un polymère à empreinte moléculaire (MIP), comprenant un polymère, qui a été produit en utilisant un ingrédient d'aliment comme cible moléculaire, au moins une des substances suivantes
de l'acide acrylique, de l'acide méthacrylique, du méthacrylate de méthyle, du 1-vinylimidazole, du 4(5)-vinylimidazole, de la 2-vinylpyridine, de l'acide itaconique, de l'acide benzoïque de p-vinyle, de l'éthylstyrène, de la (2-méthacrylamido)-6-méthylpyridine, de l'acide 2-acrylamido-2-méthyle-1-propanesulfonique, de l'acide p-vinylphénylboronique, du p-vinylbenzylamine, du 2-(p-vinylphényle)-1,3-propandiol, du p-vinylbenzylalcool, du p-vinylphénylcarbonyl, de l'acide benzoïque de p-vinyle étant utilisée comme monomère, et au moins une des substances suivantes
du diméthacrylate d'éthylène glycol (EGDMA), du para-divinylbenzène (p-DVB), du N,N'-méthylènediacrylamide, du N,N'-1,4-phénylènediacrylamide, du N,O-bisacrylol-L-phénylalaninol, du triméthacrylate de triméthylolpropane (TRIM), du triacrylate de pentaérythritol et du tétra-acrylate de pentaérythritol
étant utilisée comme crosslinker,
dans laquelle le MIP sélectivement lie à l'ingrédient d'aliment utilisé à l'origine comme cible moléculaire, pour l'utilisation dans un procédé de contrôle d'authenticité d'un analyte à partir d'un aliment par spectroscopie de masse isotopique (IRMS) ou une spectroscopie à cavité optique (CRDS) pour déterminer le ratio isotopique, choisi à partir de ¹H/²H, ¹³C/¹²C, ¹⁶O/¹⁷O/¹⁸O et/ou ¹⁵N/¹⁴N.

11. Cartouche préremplie selon la revendication 10, **caractérisé en ce qu'**au moins deux des substances suivantes
de l'acide acrylique, de l'acide méthacrylique, du méthacrylate de méthyle, du 1-vinylimidazole, du 4(5)-vinylimidazole, de la 2-vinylpyridine, de l'acide itaconique, de l'acide benzoïque de p-vinyle, de l'éthylstyrène, de la (2-méthacrylamido)-6-méthylpyridine, de l'acide 2-acrylamido-2-méthyle-1-propanesulfonique, de l'acide p-vinylphénylboronique, du p-vinylbenzylamine, du 2-(p-vinylphényle)-1,3-propandiol, du p-vinylbenzylalcool, du p-vinylphénylcarbonyl, de l'acide benzoïque de p-vinyle ont été utilisées comme des monomères pour produire le MIP.

12. Cartouche préremplie selon la revendication 10, **caractérisé en ce que** la cible est
du saccharose, du glucose ou du fructose, du maltose, du sorbitol, de la vanilline, du benzaldéhyde, du butyrate d'éthyle, de la gamma-octalactone, de la delta-décalactone, de la gamma-décalactone, du trans-2-hexénal, du 1-hexanol, du trans-2-hexanol, de l'acétate d'hexyle, du 3-(méthylthio)-propionate d'éthyle, du 3-(méthylthio)-propionate de méthyle, de l'alpha-ionone, du 2,4-décadiénoate d'éthyle, du hexanoate d'hexyle, du nootkatone, du décanal, de la 3(2H)-furanone de furanéol(4-hydroxy-2,5-diméthyle), de la 3(2H)-furanone de mésifurane(2,5-diméthyle-4-méthoxy), de l'acide ascorbique, de l'acide déshydroascorbique, de l'acide malique, de l'acide citrique, de l'acide isocitrique, de l'acide tartarique, des acides aminés, du glycérol, du phosphate, de la caféine, ou est présent en forme dérivée.
